# NEUE EUROPÄISCHE PATENTSCHRIFT

(11) **EP 1 485 342 B2**
(45) Veröffentlichungstag und Bekanntmachung des Hinweises auf die Entscheidung über den Einspruch: **04.07.2018**
(45) Hinweis auf die Patenterteilung: 18.05.2011
(21) Anmeldenummer: 03711919.5
(22) Anmeldetag: 04.03.2003
(51) Int. Cl.: C07C 67/54, C07C 51/44, C07C 51/46, C07C 69/54, C07C 57/07

(54) **VERFAHREN ZUR REINIGUNG VON BODENKOLONNEN, DIE ZUR REKTIFIKATIVEN BEHANDLUNG VON (METH) ACRYLSÄURE UND/ODER DEREN ESTER ENTHALTENDEN FLÜSSIGKEITEN VERWENDET WORDEN WAREN**
METHOD FOR CLEANING PLATE COLUMNS USED FOR RECTIFYING LIQUIDS CONTAINING (METH)ACRYLIC ACID AND/OR THE ESTERS THEREOF
PROCEDE DE NETTOYAGE DE COLONNES A PLATEAUX UTILISEES POUR LE TRAITEMENT RECTIFICATIF DE LIQUIDES CONTENANT DE L'ACIDE (METH)ACRYLIQUE ET/OU SES ESTERS

(30) Priorität: 13.03.2002 DE 10211273
(43) Veröffentlichungstag der Anmeldung: 15.12.2004
(73) Patentinhaber: BASF SE, 67056 Ludwigshafen am Rhein (DE)
(72) Erfinder: SCHRÖDER, Jürgen, 67071 Ludwigshafen (DE); MÜLLER-ENGEL, Klaus, Joachim, 76297 Stutensee (DE); SCHLIEPHAKE, Volker, 67105 Schifferstadt (DE); HAMMON, Ulrich, 68163 Mannheim (DE); DIEHL, Volker, 67158 Ellerstadt (DE); JÄGER, Ulrich, 67354 Römerberg (DE)
(86) Internationale Anmeldenummer: PCT/EP2003/002186
(87) Internationale Veröffentlichungsnummer: WO 2003/076385

(56) Entgegenhaltungen:
- WO-A-01/51159

## Beschreibung

Vorliegende Erfindung betrifft ein Verfahren zur Reinigung von Bodenkolonnen, die zur rektifikativen Behandlung von (Meth)acrylsäure und/oder deren Ester enthaltenden Flüssigkeiten verwendet worden waren, bei dem man in der Bodenkolonne von oben nach unten eine basische Flüssigkeit fördert.

(Meth)acrylsäure steht in dieser Schrift als verkürzte Schreibweise für Acrylsäure oder Methacrylsäure. Sie und ihre Ester sind wertvolle Ausgangsverbindungen zur Herstellung von durch radikalische Polymerisation erhältlichen Polymerisaten, die beispielsweise als Klebstoffe Verwendung finden.

(Meth)acrylsäure selbst wird vornehmlich durch heterogen katalysierte Gasphasenoxidation der entsprechenden Alkene, Alkane bzw. der korrespondierenden α,β-ethylenisch ungesättigten Aldehyde industriell hergestellt. Dabei wird jedoch keine reine (Meth)acrylsäure gebildet. Vielmehr entsteht ein Produktgasgemisch, aus welchem die (Meth)acrylsäure abgetrennt werden muß. Üblicherweise wird dazu die (Meth)acrylsäure in einem Lösungsmittel absorbiert und nachfolgend über verschiedene Rektifikationsstufen, gegebenenfalls unter Zusatz azeotroper Schleppmittel, vom Absorptionsmittel und darin neben (Meth)acrylsäure absorbiert enthaltenen Nebenkomponenten abgetrennt. Alternativ kann das Produktgasgemisch auch fraktionierend kondensiert und das dabei gewonnene (Meth)acrylsäure enthaltende Kondensat rektifikativ aufgearbeitet werden.

Ester der (Meth)acrylsäure werden großtechnisch in der Regel durch direkte Veresterung von (Meth)acrylsäure mit Alkoholen, z.B. Alkanolen, in Gegenwart von starken Säuren und gegebenenfalls eines Schleppmittels zur Entfernung des Veresterungswassers oder durch Umesterung von (Meth)acrylsäurestern mit geeigneten Alkoholen, z.B. Alkanolen, hergestellt. Die Abtrennung des Zielesters aus dem Produktgemisch erfolgt üblicherweise ebenfalls vorwiegend rektifikativ (vgl. z.B. EP-A 10 33 359, DE-A 19 746 688, DE-A 19 536 179 und Kirk Othmer, Encyclopedia of Chemical Technology, 4. Ed., Vol. 1, Seiten 301 - 302).

Als Rektifikationskolonnen werden sowohl im Fall der vorgenannten rektifikativen Abtrennung der (Meth)acrylsäure als auch ihrer Ester in der Regel Bodenkolonnen, d.h. Kolonnen, die als Einbauten Böden enthalten, verwendet.

Die Böden einer Bodenkolonne sind sowohl für den in der Bodenkolonnne aufsteigenden Dampf als auch für die in der Bodenkolonne rücklaufende Flüssigphase durchlässig gestaltet. Zwischen aufsteigendem Dampf und rücklaufender Flüssigphase findet insbesondere auf den Böden infolge des gestörten Gleichgewichts ein Wärme- und Stoffaustausch statt, der letztlich die in der Kolonne gewünschte Auftrennung bedingt.

Nachteilig an der rektifikativen Abtrennung von (Meth)acrylsäure und/oder ihrer Ester ist, daß es sich bei der Rektifikation einerseits um ein thermisches Trennverfahren und bei den (Meth)acrylverbindungen andererseits um Verbindungen mit hohem Siedepunkt und gleichzeitig ausgeprägter Neigung zu radikalischer Polymerisation, insbesondere unter der Einwirkung von Wärme, handelt. Typische Rektifikationstemperaturen liegen in der Regel oberhalb von 100°C. Dies gilt auch für Rektifikationen unter vermindertem Druck.

Somit sind die (Meth)acrylverbindungen bei Rektifikationen Temperaturbelastungen ausgesetzt, die leicht eine unerwünschte Polymerisation auslösen können. Die Bildung von unerwünschtem Polymerisatbelag, der im Extremfall die Bodenkolonne zu verstopfen und undurchlässig zu machen vermag, ist in der Regel die Folge. Zwar versucht man in der Praxis die unerwünschte radikalische Polymerisation durch Zusatz geeigneter Polymerisationsinhibitoren zu verhindern, doch ist auch damit eine vollständige Eliminierung der Polymerisatbildung nicht erreichbar. D.h., nach mehreren Wochen Laufzeit muß die Bodenkolonne in der Regel von Polymerisat befreit, d.h. gereinigt werden.

Aus der DE-A 19 746 688, der DE-A 19 536 179 und der EP-A 10 33 359 ist ein Verfahren zur Reinigung von Bodenkolonnen, die zur rektifikativen Behandlung von (Meth)acrylsäure und/oder deren Ester enthaltenden Flüssigkeiten verwendet worden waren, bekannt, bei dem man in der Bodenkolonne von oben nach unten eine basische Flüssigkeit fördert.

Nachteilig an dieser Verfahrensweise ist jedoch, daß die mit ihr erzielte Reinigungsgeschwindigkeit nicht voll zu befriedigen vermag.

Die WO 01/51159 betrifft ein Verfahren zum Reinigen von Trennkolonnen, durch die wässrige Polymerisatdispersionen oder Polymerisatsuspensionen im Anschluß an die Polymerisationsreaktionen zum Entfernen von leichtflüchtigen Bestandteilen geleitet wurden. Sie vermögen als Lösung der erfindungsgemäßen Aufgabe jedoch nicht vollständig zu befriedigen.

Die Aufgabe der vorliegenden Erfindung bestand daher darin, ein verbessertes Reinigungsverfahren zur Verfügung zu stellen.

Demgemäß wurde ein Verfahren zur Reinigung von Bodenkolonnen, die zur rektifikativen Reinigung von (Meth)acrylsäure und/oder deren Ester enthaltenden Flüssigkeiten verwendet worden waren und bei dem man in der Bodenkolonne von oben nach unten eine basische Flüssigkeit fördert, gefunden das dadurch gekennzeichnet ist, daß im Gegenstrom zur basischen Flüssigkeit Luft so durch die Bodenkolonne geführt wird, daß während der Reinigung der Unterschied zwischen dem Druck in der Gasphase unmittelbar unterhalb des untersten Bodens der Bodenkolonne und dem Druck in der Gasphase unmittelbar oberhalb des obersten Bodens der Bodenkolonne, geteilt durch die Anzahl der in der Kolonne befindlichen Böden, 0,5 bis 5 mbar pro Boden beträgt.

Bevorzugt wird die Luft mit einer solchen Geschwindigkeit durch die Bodenkolonne geführt, daß der vorgenannte mittlere Druckverlust zwischen zwei aufeinanderfolgenden Trennböden 1 bis 5 mbar und besonders bevorzugt 2 bis 4 mbar beträgt.

Bei den in der Bodenkolonne befindlichen Trennböden kann es sich z.B. um Dual-Flow-Böden, um Siebböden, um Ventilböden, um Thormann-Böden, um Tunnelböden und/oder um Glockenböden handeln.

Zur Förderung der Luft können Verdichter (Kompressoren) und/oder Vakuumpumen verwendet werden.

Als basische Flüssigkeiten können für das erfindungsgemäße Verfahren alle diejenigen eingesetzt werden, die auch die DE-A 19 746 688, die DE-A 19 536 179 und die EP-A 10 33 359 empfehlen.

Das sind im besonderen wäßrige Alkali- und/oder Erdalkalihydroxid- und/oder -oxidlösungen, vor allem die wäßrigen Lösungen von NaOH, KOH und Ca (OH)₂. In der Regel weist dabei die wäßrige Lösung einen gelösten Salzgehalt von 0,01 bis 30 Gew.-%, vorzugsweise von 0,5 bis 10 Gew.-% auf.

Gemäß einer vorteilhaften Weiterbildung der Erfindung wird der vorgenannten basischen wäßrigen Alkalilösung im Verhältnis von > 0:1 bis 2:1 (Gewichtsverhältnis von Neutralsalz zu Hydroxid und/oder Oxid) ein im wesentlichen ph-neutrales (bezogen auf seine wäßrige Lösung) Alkali- und/oder Erdalkalisalz zugesetzt. Hierfür eignen sich besonders die den hydroxidischen/oxidischen Verbindungen entsprechenden Sulfate, Acetate, Oxalate, Carbonate, Hydrogensulfate, Hydrogencarbonate und/oder andere Salze. Durch einen solchen Zusatz läßt sich das Lösungsverhalten der basischen Lösung für das erfindungsgemäße Verfahren weiter verbessern.

Anstelle basischer wäßriger Lösungen können erfindungsgemäß aber auch basisch reagierende polare organische Lösungsmittel wie Amine oder Amide, bevorzugt Acetamide, besonders bevorzugt Monoacetamid (CH₃CONH₂) als basische Flüssigkeit eingesetzt werden. Weitere basische Flüssigkeiten die erfindungsgemäß eingesetzt werden können sind Monomethylacetamid (CH₃CON(CH₃)H), Dimethylacetamid (CH₃CON (CH₃)₂) sowie Dimethylformamid (HCON(CH₃)₂).

Die Temperaturen, bei denen die erfindungsgemäße Spülung durchgeführt wird, werden im wesentlichen vom Siedepunkt der eingesetzten basischen Flüssigkeit bestimmt, da für alle genannten basischen Flüssigkeiten gilt, daß ihr Lösevermögen mit steigender Temperatur zunimmt. Die optimale Einsatztemperatur für die wäßrigen Alkali- und/oder Erdalkalihydroxidlösungen liegt bei > 80°C bis ca. 115°C bei Normaldruck, vorzugsweise bei 90°C bis 110°C. Für die beschriebenen Amide liegt die optimale Einsatztemperatur jeweils 10 bis 1°C unterhalb des Siedepunktes dieser Substanzen.

Das erfindungsgemäße Verfahren kann sowohl in regelmäßigen Zeitabständen wie auch nach Feststellung einer bestimmten Polymerisatbildung durchgeführt werden.

Die in der erfindungsgemäß zu reinigenden Bodenkolonne zuvor rektifikativ behandelten (Meth)acrylsäure und/oder deren Ester enthaltenden Flüssigkeiten können ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% (Meth)acrylsäure und/oder deren Ester enthalten. Bei den Estern kann es sich dabei um die Ester mit Monohydroxy- und/oder Polyhydroxyalkoholen handeln.

Insbesondere umfassen die (Meth)acrylsäureester die Ester der (Meth)acrylsäure mit Alkanolen (C₁- bis C₁₂-, bevorzugt C₁- bis C₈-) und/oder Alkandiolen. Das sind vor allem Methylacrylat, Methylmethacrylat, Ethylacrylat, Ethylmethacrylat, n-Butylacrylat, tert.-Butylacrylat, tert.-Butylmethacrylat sowie 2-Ethylhexylacrylat, aber auch die Ester des Dimethylaminoethanols.

Zur möglichst weitgehenden Unterdrückung von Polymerisatbildung während der Rektifikation zugesetzte Polymerisationsinhibitoren sind z.B. Stabilisatoren wie Phenothiazin, Hydrochinonmonomethylether oder Hydrochinon.

Der Vorteil des erfindungsgemäßen Verfahrens besteht darin, daß die Polymerisatbeseitigung einen geringeren Zeitaufwand erfordert. Im Endergebnis wird so eine kurze Unterbrechungsdauer des Rektifikationsverfahrens sowie eine vollständige Entfernung der Polymerisat-Niederschläge erreicht.

Vorab des erfindungsgemäßen Verfahrens und nach Durchführung des erfindungsgemäßen Verfahrens wird die Bodenkolonne in fachmännischer Weise mit Wasser gespült. Dabei ist es wie beim erfindungsgemäßen Verfahren in gleicher Weise vorteilhaft im Gegenstrom zum Spülwasser ein Gas durch die Bodenkolonne zu fördern. Abschließend wird die Bodenkolonne getrocknet und weiterbetrieben.

Polymerisatbelag weist sich beim erfindungsgemäßen Verfahren in der Regel durch einen zunehmenden Druckverlust beim Betrieb der Bodenkolonne aus.

Damit Bodenkolonnen nach dem erfindungsgemäßen Verfahren gut zu reinigen sind, sind sie in zweckmäßiger Weise mit einer Spülleitung ausgerüstet. Diese ermöglicht den Transport der basischen Spülflüssigkeit, die beispielsweise im Verdampfer der Kolonne erhitzt wird, zum Kopf der Kolonne. Üblicherweise wird die Spülflüssigkeit über die Rücklaufleitung der Rektifikationskolonne zugeführt.

Das erfindungsgemäße Verfahren kann bei Normaldruck, Überdruck oder bei reduziertem Druck durchgeführt werden.

Der Druck in der Gasphase "unmittelbar" unterhalb des untersten bzw. oberhalb des obersten Bodens der Bodenkolonne soll in dieser Schrift bedeuten, daß die Meßstelle nicht mehr als 15 cm unterhalb des untersten und wenigstens 25 cm oberhalb des obersten Bodens liegen soll. Die Druckmessung kann z.B. via offene Anbohrung erfolgen, bei der ein Meßumformer über einen Wandstutzen mit der Kolonne verbunden ist.

Die erfindungsgemäße Verfahrensweise kann selbstredend auch für Kolonnen angewendet werden, die andere Einbauten als Böden (z.B. Raschig Ringe, Pallringe oder Packungen) enthalten und in denen (Meth)acrylverbindungen haltige Flüssigkeiten rektifiziert wurden und/oder in denen aus (Meth)acrylmonomeren haltigen Dämpfen absorbiert wurde.

Es ist ferner auch dann anwendbar, wenn die entsprechende Kolonne im Verlauf einer Absorption von (Meth)acrylverbindungen aus der Gasphase mit Polymerisat verschmutzt worden war.

Insgesamt wird die Vorteilhaftigkeit des erfindungsgemäßen Verfahrens darauf zurückgeführt, daß sich unter den gegebenen Randbedingungen in der in der Kolonne von oben nach unten geführten Spülflüssigkeit Flüssigkeitsstrudel ausbilden, vor allem auf den Kolonnenböden, die die beschleunigte Reinigung bewirken.

### Beispiel

In einer Bodenkolonne (Material: ein Edelstahl mit der Werkstoffnummer 1.4571 entsprechend der Norm DIN EN 10020) mit 3,8 m Durchmesser und einer Länge von 32 m wurde aus der wie nachfolgend beschrieben zusammengesetzten Flüssigkeit (Zuflußmenge = 114 Tonnen/h) über die Zulaufleitung der Kolonne Acrylsäure rektifikativ abgetrennt.

Die Flüssigkeit enthielt:

| | |
|---|---|
| 17 Gew.-% | Acrylsäure, |
| 0,02 Gew.-% | Wasser, |
| 0,0015 Gew.-% | Acrolein, |
| 0,0015 Gew.-% | Acrylacrylat, |
| 0,01 Gew.-% | Furfural, |
| 0,027 Gew.-% | Essigsäure, |
| 0,2 Gew.-% | Benzaldehyd, |
| 0,003 Gew.-% | Propionsäure, |
| 0,032 Gew.-% | Maleinsäureanhydrid, |
| 58 Gew.-% | Diphyl, |
| 17,0 Gew.-% | Dimethylphthalat, |
| 3 Gew.-% | Acryloylpropionsäure und |
| 0,02 Gew.-% | Phenothiazin. |

Die Bodenkolonne enthielt 45 Dual-Flow-Böden (Material: ein Edelstahl mit der Werkstoffnummer 1.4571 entsprechend der Norm DIN EN 10020). 37 Böden befanden sich oberhalb der Zulaufstelle und 8 Böden befanden sich unterhalb der Zulaufstelle der die Acrylsäure enthaltenden Flüssigkeit. Die Dual-Flow-Böden oberhalb des Zulaufs wiesen Bohrlöcher des Durchmessers 25 mm und die Dual-Flow-Böden unterhalb des Zulaufs wiesen Bohrlöcher des Durchmessers 50 mm auf (jeweils innen gemessen). Die Acrylsäure haltige Flüssigkeit wurde in eine 99,6 gew.-%ige Acrylsäure, ein Gemisch aus den leichter als Acrylsäure siedenden Komponenten und ein Gemisch aus schwerer als Acrylsäure siedenden Komponenten, das weniger als 0,5 Gew.-% Acrylsäure enthielt, aufgetrennt. Der Abstand der Dual-Flow-Böden betrug über die gesamte Bodenkolonne homogen 400 mm. Die Temperatur am Kopf der Kolonne betrug 80°C, der Druck am Kolonnenkopf betrug 105 mbar und das Rücklaufverhältnis (ml Rücklaufflüssigkeit zu ml Flüssigkeitsentnahme) betrug 1,3. Die Temperatur am Sumpf der Kolonne betrug 193°C und der Druck am Kolonnensumpf lag bei 230 mbar. Der Rücklauf der Kolonne wurde mit Phenothiazin so stabilisiert, daß die über Seitenabzug (auf Boden 35, von unten gerechnet) entnommene 99,6 gew.-%ige Acrylsäure 250 Gew.-ppm PTZ enthielt. Das PTZ wurde in solchermaßen abgetrennter Acrylsäure gelöst (1,5 gew.-%ige Lösung) zugesetzt.

Nach einer Laufzeit von 21 Tagen wurde die Rektifikationskolonne abgeschaltet, entleert und anschließend zwei Stunden mit Wasser der Temperatur 30°C gespült. Das Spülwasser wurde der Bodenkolonne über deren obere Rücklaufleitung im freien Fall zugeführt und über selbige im Kreis gepumpt (300 m³/l). Das gebrauchte Spülwasser wurde der Kolonne nach beendeter Spülung entnommen. Das darin enthaltene Diphyl/Dimethylphthalat-Gemisch konnte durch Wasserdampfdestillation rückgewonnen werden.
Anschließend wurde die Bodenkolonne inspiziert.

Im Bereich unterhalb des Seitenabzugs (insbesondere im Bereich von Boden 30 bis Boden 35) befanden sich ca. 250 kg Polymerisat. Das Polymerisat haftete sowohl auf den Böden (ca. 60 %) als auch an der Bodenunterseite (ca. 40 %).

Anschließend wurde mit einer 5 gew.-%igen wäßrigen Natronlauge gespült. Die wäßrige Natronlauge wurde der Bodenkolonne über die Zulaufleitung im freien Fall zugeführt und über selbige im Kreis gepumpt (300 m³/h). Der Sumpfverdampfer der Bodenkolonne war dabei eingeschaltet, um die Temperatur der Natronlauge auf 90 bis 95°C einzustellen.

Nach einer Stunde wurde der Zulauf (im freien Fall) auf die obere Rücklaufleitung umgestellt und die Natronlauge über selbige weitere sechs Stunden im Kreis gepumpt (300 m³/h). Während der Gesamtdauer der Natronlaugespülung wurden unterhalb des ersten Bodens 600 m³/h Umgebungstemperatur aufweisende Luft in die Kolonne geführt. Der mittlere Gasphasen-Differenzdruck über alle Böden betrug 2 mbar/Boden. Nach beendeter Spülung wurde die gebrauchte Spüllauge der Bodenkolonne entnommen. Das darin enthaltene Diphyl/Dimethylphthalat-Gemisch konnte durch Wasserdampfdestillation rückgewonnen werden. Anschließend wurde die Bodenkolonne inspiziert. Das Polymerisat war bis auf geringfügige Restmengen (<5 kg) abgelöst.

### Vergleichsbeispiel

Es wurde wie im Beispiel verfahren. Nach der Wasserspülung wurde die Rektifikationskolonne inspiziert. Im Bereich unterhalb des Seitenabzugs befanden sich ca. 200 kg Polymerisat. Das Polymerisat haftete sowohl auf den Böden (ca. 50 %) als auch an der Bodenunterseite (ca. 50 %).

Anschließend wurde wie im Beispiel mit Natronlauge gespült, wobei jedoch auf eine Luftzufuhr verzichtet wurde. Der mittlere Gasphasen-Differenzdruck über alle Böden betrug « 0,5 mbar/Boden. Nach beendeter Natronlaugespülung wurde die Rektifikationskolonne inspiziert. Im Bereich unterhalb des Seitenabzugs befanden sich noch ca. 80 kg Polymerisat. Das Polymerisat befand sich sowohl auf den Böden (ca. 40 %) als auch an der Bodenunterseite (ca. 60 %).

Anschließend wurde die Natronlaugenspülung wie im Beispiel, d.h. mit Luftzufuhr, wiederholt. Der mittlere Gasphasendifferenzdruck über alle Böden betrug 2,2 mbar/Boden. Nach der zweiten Natronlaugespülung wurde die Bodenkolonne erneut inspiziert. Das Polymerisat war bis auf geringfügige Restmengen (<5 kg) abgelöst.

Die Druckmessung erfolgte sowohl im Beispiel als auch im Vergleichsbeispiel jeweils 10 cm unterhalb des untersten bzw. 30 cm oberhalb des obersten Bodens via offene Anbohrung (Abstand zur Oberkante der Anbohrung), wobei ein Meßumformer über einen Wandstutzen mit der Kolonne verbunden war.

## Patentansprüche

1. Verfahren zur Reinigung von Bodenkolonnen, die zur rektifikativen Behandlung von (Meth)acrylsäure und/oder deren Ester enthaltenden Flüssigkeiten verwendet worden waren, bei dem man in der Bodenkolonne von oben nach unten eine basische Flüssigkeit fördert, **dadurch gekennzeichnet, dass** im Gegenstrom zur basischen Flüssigkeit Luft so durch die Bodenkolonne geführt wird, dass während der Reinigung der Unterschied zwischen dem Druck in der Gasphase unmittelbar unterhalb des untersten Bodens der Bodenkolonne und dem Druck in der Gasphase unmittelbar oberhalb des obersten Bodens der Bodenkolonne, geteilt durch die Anzahl der in der Kolonne befindlichen Böden 0,5 bis 5 mbar pro Boden beträgt.

2. Verfahren nach Anspruch 1, **dadurch gekennzeichnet, dass** während der Reinigung der Unterschied zwischen dem Druck in der Gasphase unmittelbar unterhalb des untersten Bodens der Bodenkolonne und dem Druck in der Gasphase unmittelbar oberhalb des obersten Bodens der Bodenkolonne, geteilt durch die Anzahl der in der Kolonne befindlichen Böden, 1 bis 5 mbar beträgt.

3. Verfahren nach Anspruch 1, **dadurch gekennzeichnet, dass** während der Reinigung der Unterschied zwischen dem Druck in der Gasphase unmittelbar unterhalb des untersten Bodens der Bodenkolonne und dem Druck in der Gasphase unmittelbar oberhalb des obersten Bodens der Bodenkolonne, geteilt durch die Anzahl der in der Kolonne befindlichen Böden, 2 bis 4 mbar beträgt.

4. Verfahren nach einem der Ansprüche 1 bis 3, **dadurch gekennzeichnet, dass** als basische Flüssigkeit eine wässrige Lösung von Natriumhydroxid verwendet wird.

## Claims

1. A process for cleaning tray columns which have been used for rectificatively treating liquids comprising (meth)acrylic acid and/or esters thereof by conveying a basic liquid downward through the tray column, which comprises passing air through the tray column in countercurrent to the basic liquid in such a manner that, during the cleaning, the difference between the pressure in the gas phase immediately below the lowermost tray of the tray column and the pressure in the gas phase immediately above the uppermost tray of the tray column divided by the number of trays in the column is from 0.5 to 5 mbar per tray.

2. The process according to claim 1, wherein, during the cleaning, the difference between the pressure in the gas phase immediately above the uppermost tray of the tray column and the pressure in the gas phase immediately below the lowermost tray of the tray column divided by the number of trays in the column is from 1 to 5 mbar.

3. The process according to claim 1, wherein, during the cleaning, the difference between the pressure in the gas phase immediately above the uppermost tray of the tray column and the pressure in the gas phase immediately below the lowermost tray of the tray column divided by the number of trays in the column is from 2 to 4 mbar.

4. The process according to any of claims 1 to 3, wherein the basic liquid used is an aqueous solution of sodium hydroxide.

## Revendications

1. Procédé de nettoyage de colonnes à plateaux utilisées pour le traitement rectificatif de liquides contenant de l'acide (méth)acrylique et/ou ses esters, selon lequel un liquide basique est mis en circulation de haut en bas dans la colonne à plateaux, **caractérisé en ce que** de l'air est mis en circulation dans la colonne à plateaux à contre-courant du liquide basique de manière à ce que, pendant le nettoyage, la différence entre la pression dans la phase gazeuse directement en-dessous du plateau le plus inférieur de la colonne à plateaux et la pression dans la phase gazeuse directement au-dessus du plateau le plus supérieur de la colonne à plateaux, divisée par le nombre de plateaux se trouvant dans la colonne, soit de 0,5 à 5 mbar par plateau.

2. Procédé selon la revendication 1, **caractérisé en ce que**, pendant le nettoyage, la différence entre la pression dans la phase gazeuse directement en-dessous du plateau le plus inférieur de la colonne à plateaux et la pression dans la phase gazeuse directement au-dessus du plateau le plus supérieur de la colonne à plateaux, divisée par le nombre de plateaux se trouvant dans la colonne, est de 1 à 5 mbar.

3. Procédé selon la revendication 1, **caractérisé en ce que**, pendant le nettoyage, la différence entre la pression dans la phase gazeuse directement en-dessous du plateau le plus inférieur de la colonne à plateaux et la pression dans la phase gazeuse directement au-dessus du plateau le plus supérieur de la colonne à plateaux, divisée par le nombre de plateaux se trouvant dans la colonne, est de 2 à 4 mbar.

4. Procédé selon l'une quelconque des revendications 1 à 3, **caractérisé en ce qu'**une solution aqueuse d'hydroxyde de sodium est utilisée en tant que liquide basique.
